# EUROPEAN PATENT SPECIFICATION

(11) **EP 3 043 829 B1**
(45) Date of publication and mention of the grant of the patent: **19.12.2018**
(21) Application number: 14843282.6
(22) Date of filing: 09.09.2014
(51) Int. Cl.: A61K 49/22, A61K 31/28

(54) **PHOTOACOUSTIC IMAGING CONTRAST AGENT COMPOSITION**
KONTRASTMITTELZUSAMMENSETZUNG FÜR PHOTOAKUSTISCHE BILDGEBUNG
COMPOSITION D'AGENT DE CONTRASTE D'IMAGERIE PHOTOACOUSTIQUE

(30) Priority: 12.09.2013 SG 201306889
(43) Date of publication of application: 20.07.2016
(73) Proprietor: Agency for Science, Technology and Research, Singapore 138632 (SG); Nanyang Technological University, Singapore 639798 (SG)
(72) Inventor: OLIVO, Malini, Singapore 138667 (SG); KONG, Kien Voon, Singapore 138667 (SG); LEONG, Weng Kee, Singapore 637371 (SG)
(74) Representative: Viering, Jentschura & Partner mbB Patent- und Rechtsanwälte
(86) International application number: PCT/SG2014/000423
(87) International publication number: WO 2015/038066

(56) References cited:
- WO-A1-2014/137291
- KIEN VOON KONG ET AL: "Bioimaging in the Mid-Infrared Using an Organometallic Carbonyl Tag", BIOCONJUGATE CHEMISTRY, vol. 18, no. 5, 1 September 2007 (2007-09-01), pages 1370-1374, XP055324448, ISSN: 1043-1802, DOI: 10.1021/bc070133a
- KONG KIEN VOON: "BIOORGANOMETALLIC CHEMISTRY OF OSMIUM CARBONYL CLUSTERS", NATIONAL UNIVERSITY OF SINGAPORE , 2008, pages Frontpg.-110, XP002768159, Retrieved from the Internet: URL:http://scholarbank.nus.edu.sg/bitstrea m/handle/10635/28214/KKV%20Thesis.pdf?sequ ence=1 [retrieved on 2017-03-13]
- KIEN VOON KONG ET AL: "Metal Carbonyl-Gold Nanoparticle Conjugates for Live-Cell SERS Imaging", ANGEWANDTE CHEMIE INTERNATIONAL EDITION, vol. 51, no. 39, 3 September 2012 (2012-09-03), pages 9796-9799, XP055183614, ISSN: 1433-7851, DOI: 10.1002/anie.201204349
- BUTLER I S (REPRINT): "FT-IR, PHOTOACOUSTIC AND MICRO-RAMAN SPECTRA OF THE DODECACARBONYLTRIRUTHENIUM (0) COMPLEXES (RU3CO12)-C-13 AND RU3(CO)12", JOURNAL OF RAMAN SPECTROSCOPY, vol. 18, no. 5, 1 August 1987 (1987-08-01) , pages 357-363, XP008183698, JOHN WILEY & SONS LTD, GB ISSN: 0377-0486, DOI: 10.1002/JRS.1250180511 [retrieved on 2005-04-14]
- LI ET AL: "The reaction of triosmium and -ruthenium clusters with bifunctional ligands", JOURNAL OF ORGANOMETALLIC CHEMISTRY, vol. 693, no. 7, 20 January 2008 (2008-01-20), pages 1292-1300, XP022524973, ELSEVIER-SEQUOIA S.A. LAUSANNE, CH ISSN: 0022-328X, DOI: 10.1016/J.JORGANCHEM.2008.01.025
- GEOFFREY P LUKE ET AL: "Biomedical Applications of Photoacoustic Imaging with Exogenous Contrast Agents", ANNALS OF BIOMEDICAL ENGINEERING, vol. 40, no. 2, 3 November 2011 (2011-11-03), pages 422-437, XP035016163, KLUWER ACADEMIC PUBLISHERS-PLENUM PUBLISHERS, NE ISSN: 1573-9686, DOI: 10.1007/S10439-011-0449-4
- ZHIYONG LAM ET AL: "High nuclearity carbonyl clusters as near-IR contrast agents for photoacoustic in vivo imaging", JOURNAL OF MATERIALS CHEMISTRY B, vol. 4, no. 22, 1 January 2016 (2016-01-01), pages 3886-3891, XP055354063, GB ISSN: 2050-750X, DOI: 10.1039/C6TB00075D
- KONG K. V. ET AL.: 'Bioimaging in the Mid-Infrared Using an Organometallic Carbonyl Tag' BIOCONJUGATE CHEM. vol. 18, no. 5, 2007, pages 1370 - 1374, XP055324448
- KONG K.V. ET AL.: 'Organometallic carbonyl clusters: a new class of contrast agents for photoacoustic cerebral vascular imaging' CHEM. COMMUN. vol. 50, 2014, pages 2601 - 2603, XP055324450

## Description

### TECHNICAL FIELD

Various embodiments relate to photoacoustic contrast agent compositions.

### BACKGROUND

Photoacoustic imaging (PAI) is a non-invasive imaging technique which generally involves flashing a laser at low energy onto a target area or region on a subject's body. The laser at low energy may penetrate deeply into the body to create a large radiated area for more detailed imaging. Rapid absorption of laser energy may expand the tissue through transient thermo-elastic expansion. This expansion creates ultrasonic acoustic waves that may be detected using ultrasound detectors of appropriate sensitivity, such as ultrasound transducers. The transducer readings may be processed and interpreted using mathematical algorithms to create two dimensional or three dimensional images of the target area to depict the tissue structure.

Researches have been carried out in search for suitable contrast agents to assist in generating images using PAI. Examples of contrast agents include organic based contrast agents, such as cyanine dyes, nanoparticles, polyhydroxy-fullerene and carbon nanotubes. However, the photoacoustic contrast that is achieved using the contrast agents may be low, resulting in poor spatial resolution of the images.

Kien Voon Kong et al. ("Bioimaging in the Mid-Infrared Using an Organometallic Carbonyl Tag", Bioconjugate Chem. 2007, vol. 18, no. 5, 1370-1374) discloses preparation of stable and water-soluble organometallic carbonyl cluster derivatives such as Os₃(CO)₁₀(µ-H)µ-S-(CH₂)₁₀-COO⁻Na⁺. The CO stretching vibrations afford strong mid-infrared signals for bioimaging via an IR microscope.

Kong Kien Voon ("Bioorganometallic Chemistry of Osmium Carbonyl Clusters", National University of Singapore, 2008, pages 1-110) discloses osmium carbonyl clusters, including Os₃(CO)₁₀(µ-H)µ-S-(CH₂)₁₀-COO⁻Na⁺, Os₃(CO)₁₂, and Os₃(CO)₁₀(µ-H)₂.

Butler I. S. ("FT-IR, Photoacoustic and Micro-Raman Spectra of the Dodecacarbonyltriruthenium (0) Complexes (Ru3(13CO)12 and Ru3(CO)12", Journal of Raman Spectroscopy, Vol. 18, No. 5, pages 357-363) discloses room-temperature FT-IR, photoacoustic and micro-Raman spectra for Ru₃(CO)₁₂.

Geoffrey P. Luke et al. ("Biomedical Applications of Photoacoustic Imaging with Exogenous Contrast Agents", Annals of Biomedical Engineering, Vol. 40, No. 2, Pages 422-437) provides an overview of exogenous contrast agents used in photoacoustic imaging.

In view of the above, there is a need for photoacoustic contrast agents that are able to exhibit improved photoacoustic contrasts.

### SUMMARY

The invention is defined by the claims. Any subject-matter falling outside the scope of the claims is provided for information purposes only.

Use of a photoacoustic imaging contrast agent composition in photoacoustic microscopy carried out in a subject at a wavelength in the visible range of 400 nm to 750 nm is provided. The composition comprises a metal carbonyl cluster compound having the general formula (I)

M₃(CO)ₓL₁₂₋ₓ (I)

wherein M at each occurrence denotes a metal independently selected from the group consisting of Fe, Ru, and Os; x is an integer from 10 to 12; and each L is independently selected from the group consisting of -H and -A-(CH₂)ₙ-COO⁻Y⁺, wherein A is selected from the group consisting of S, O, C and N; n is an integer from 1 to 10; and Y is any cation.

### BRIEF DESCRIPTION OF THE DRAWINGS

The invention will be better understood with reference to the detailed description when considered in conjunction with the examples and the accompanying drawings, in which:
**FIG. 1A** shows UV-vis spectra of organometallic compounds M₃(CO)₁₂ (M = Fe, Ru or Os, **1 a-c**) used for photoacoustic (PA) study. Single-walled carbon nanotubes (SWNT) were used for comparison. Y-axis: absorbance; x-axis: wavelength (nm). **FIG. 1B** shows false color images representing the relative PA signal strengths for **1 a-c**, SWNT, 40 % ethanol, and water.
**FIG. 2A** shows synthetic scheme for water-soluble cluster [Os₃(CO)₁₀(µ-H)(µ-S(CH₂)₂COO)]⁻Na⁺ (2). Short lines represent carbonyl ligands. **FIG. 2B** shows (3-(4,5-dimethylthiazol-2-yl)-5 -(3 -carboxymethoxyphenyl)-2-(4-sulfophenyl)-2H-tetrazolium) (MTS) assay for **2,** at concentrations of 0 µM, 0.01 µM, 0.05 µM, 0.1 µM, 0.5 µM, 1 µM, 2.5 µM, 5 µM, 10 µM, 12.5 µM, and 30 µM. Y-axis: viability (%); x-axis: concentration (µM).
**FIG. 3** shows PA signal of **2** at different concentrations, where **FIG. 3A** shows plot of PA B-scan signal strength vs concentrations of **2.** **FIG. 3B** shows false-colour images representing the relative PA signal intensity. PA B-scan images were performed from a polyethylene tube filled with solutions of **2** taken at 410 nm, at concentrations of 30 µM, 5 µM, 15 µM, 2.5 µM, 12.3 µM, 0 µM (H₂O), and 1.0 µM.
**FIG. 4** is a schematic diagram showing experimental setup of the functional photoacoustic microscopy.
**FIG. 5** depicts PA imaging of cortical blood vessels. **FIG. 5A** is a plot of PA signal strength before, and 20 min after, administration of a 30 µM aqueous solution of **2,** and **FIG. 5B** is a photograph of the rat cortical blood vessels, with the superior sagittal sinus (SSS) indicated. The ROI cross-section location is indicated by the blue arrow. **FIG. 5C** is PA B-scan images acquired before, and 20 min after, administration of a 30 µM aqueous solution of **2.** The SSS is highlighted with a dashed oval.

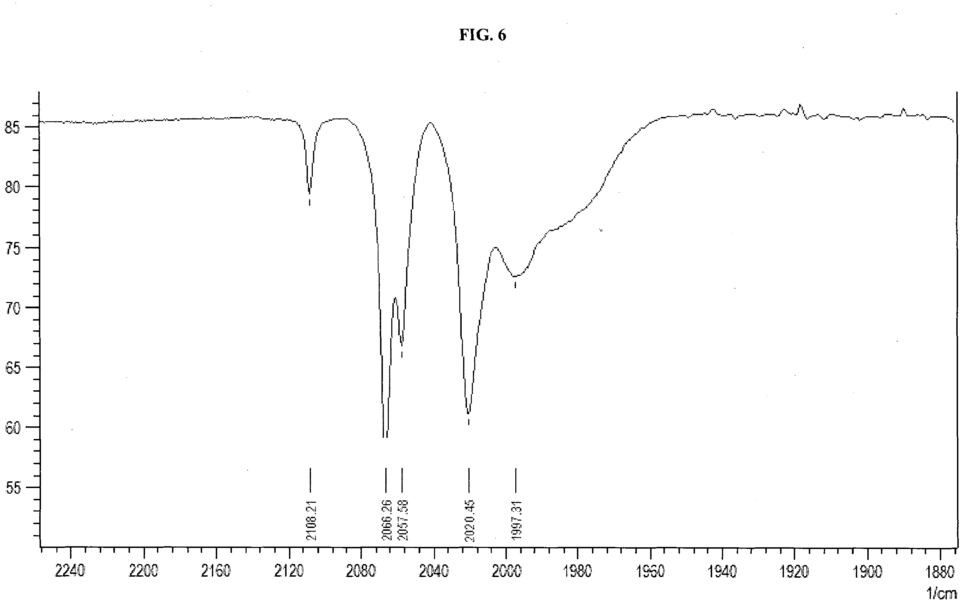

**FIG. 6** is a graph showing infra-red (IR) spectroscopy spectrum of Os₃(CO)₁₀(µ-H)µ-S(CH₂)₂COOH. Peaks at 2108.21, 2066.26, 2057.58, 2020.45, and 1997.31 are indicated.

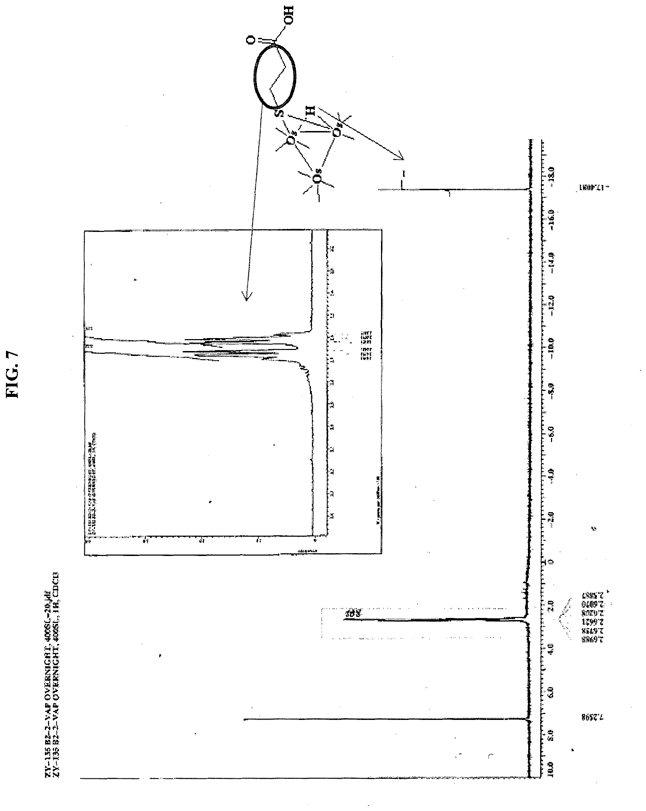

**FIG. 7** is a graph showing nuclear magnetic resonance spectroscopy (NMR) spectroscopy spectrum of Os₃(CO)₁₀(µ-H)µ-S(CH₂)₂COOH. X-axis range from 10.0 to -18.0, increments of 2 per interval. Peaks at 7.2598, 2.6988, 2.6758, 2.6621, 2.6208, 2.6070, 2.5887, and -17.4081 are indicated. Inset of **FIG. 7** is an expanded view of the spectrum in the range of 3.4 to 2.2.

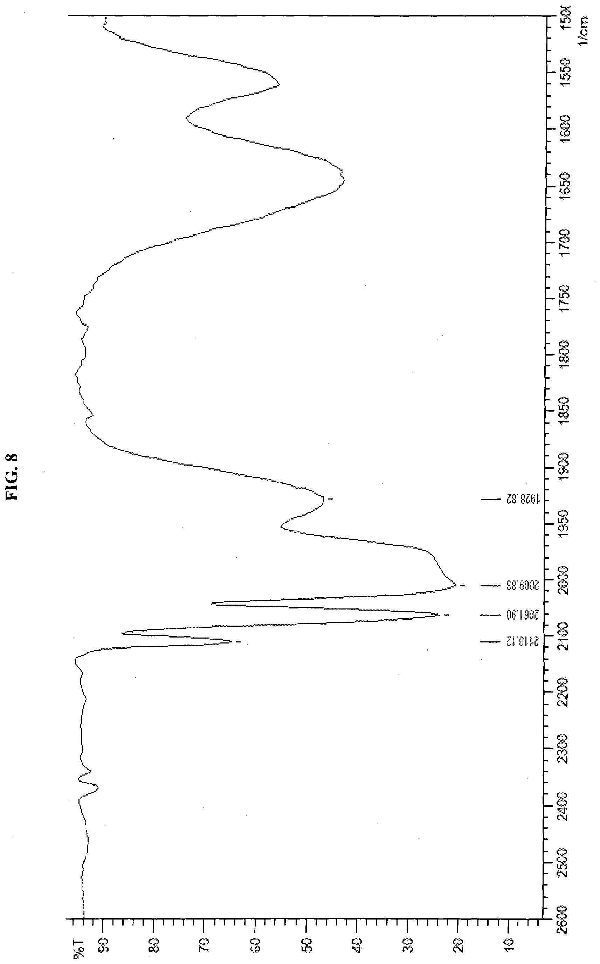

**FIG. 8** is a graph showing solid IR spectrum of Os salt. Peaks at 2110.12, 2061.90, 2009.83, and 1928.82 are indicated.

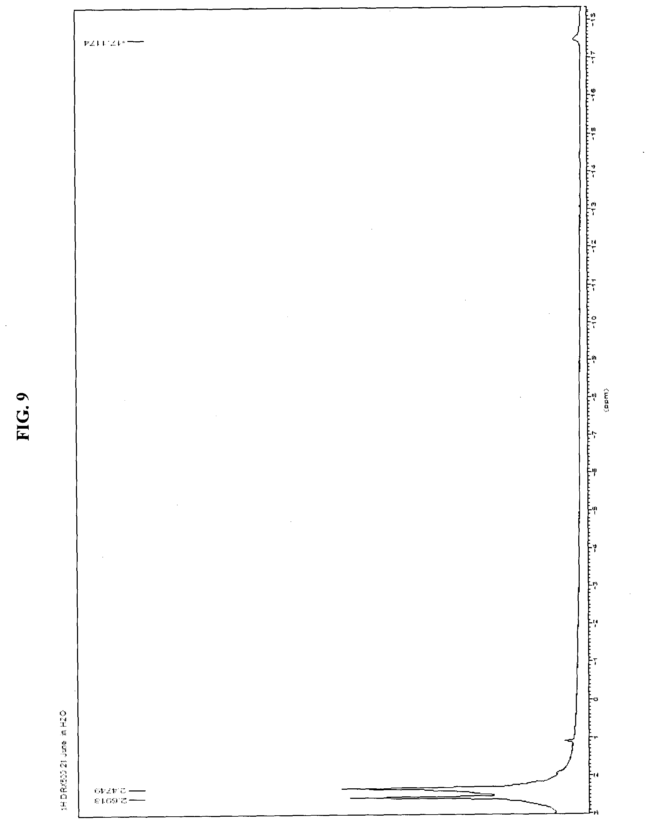

**FIG. 9** is a graph showing NMR spectrum of Os salt. X-axis labels range from 3 ppm to -18 ppm, increments of 1 ppm per interval. Peaks at 2.6913, 2.4749, and -17.1174 are indicated.

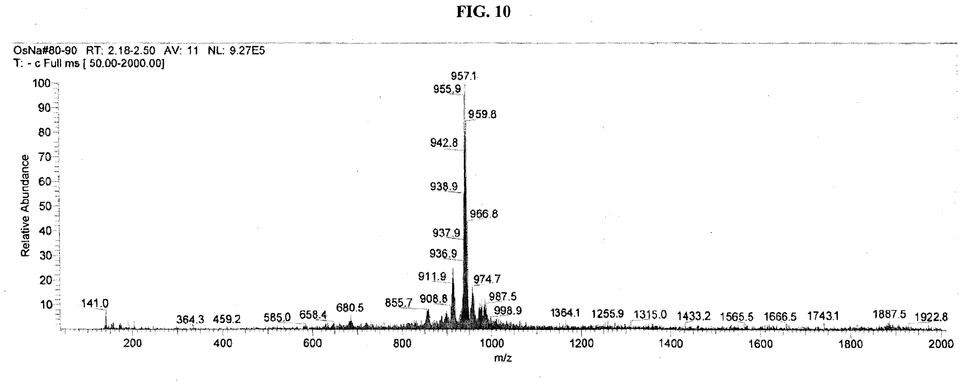

**FIG. 10** is a graph showing negative ion electrospray mass spectrum of Os salt in aqueous solution.

### DETAILED DESCRIPTION

Photoacoustic imaging is based on the mechanism that electromagnetic waveforms, such as radio frequency (rf) or optical waves, may be absorbed by a material, to result in local heating and thermoelastic expansion. The thermoelastic expansion may, in turn, produce megahertz ultrasonic waves in the material, thereby generating a photoacoustic signal. Accordingly, the term "photoacoustic imaging" as used herein refers to signal generation caused by an electromagnetic pulse, with absorption and expansion of a photoacoustic imaging contrast agent, followed by acoustic detection, where the photoacoustic imaging contrast agent absorbs the light energy and converts it to thermal energy that generates the photoacoustic signal.

A photoacoustic imaging contrast agent composition comprising a metal carbonyl cluster compound is disclosed herein. Advantageously, the metal carbonyl cluster compound offers multiple sites for attachment of various ligands as property modifiers. Further, by conjugation to a myriad of bioactive agents such as proteins, antibodies, and drugs, molecular imaging and therapeutic monitoring may be carried out.

As used herein, the term "metal carbonyl cluster" refers to metal cluster compounds comprising carbon monoxide in complex combination with metal atoms, wherein the metal atoms in the metal carbonyl cluster are held together entirely or at least substantially by bonds between metal atoms.

The metal carbonyl cluster compound has general formula (I)

M₃(CO)ₓL₁₂₋ₓ (I),

wherein M at each occurrence denotes a metal independently selected from the group consisting of Fe, Ru, and Os; x is an integer from 10 to 12; and each L is independently selected from the group consisting of -H and -A-(CH₂)ₙ-COO⁻Y⁺, wherein A is selected from the group consisting of S, O, C and N; n is an integer from 1 to 10; and Y is any cation. CO in formula (I) denotes a carbonyl ligand.

In one embodiment, M is Os.

The metal carbonyl cluster compound may comprise more than one metal. For example, M in the general formula M₃(CO)ₓL₁₂₋ₓ mentioned above may be represented by (Mₐ)₂M_{b}, Mₐ(M_{b})₂, (M_{b})₂M_{c}, M_{b}(M_{c})₂, (Mₐ)₂M_{c}, Mₐ(M_{c})₂, or MₐM_{b}M_{c}, where Mₐ, M_{b} and M_{c} denote different metals. In such embodiments, the metal carbonyl compound may have general formula MₐM_{b}M_{c}(CO)ₓL₁₂₋ₓ, wherein Mₐ, M_{b}, and Mₑ denote different metals, and CO, L, and x having the same definitions as that mentioned above.

In general formula (I), x is an integer from 10 to 12, such as 10, 11, or 12. In various embodiments, x is 10.

The metal carbonyl cluster compound may contain one or more different ligands besides carbonyl ligands, as represented by L in general formula (I). Each L is independently selected from the group consisting of -H and -A-(CH₂)ₙ-COO⁻Y⁺, wherein A is selected from the group consisting of S, O, C and N; n is an integer from 1 to 10; and Y is any cation.

In various embodiments, A is S.

In various embodiments, n is an integer from 1 to 6, such as 1, 2, 3, 4, 5, or 6. In specific embodiments, n is 2.

In various embodiments, Y is selected from the group consisting of an alkali metal and NH₄. For example, Y may be lithium (Li), sodium (Na), potassium (K), rubidium (Rb), cesium (Cs), or NH₄. In some embodiments, Y is selected from the group consisting of Na and K. In specific embodiments, Y is Na.

In specific embodiments, the metal carbonyl cluster compound is Os₃(CO)₁₀(µ-H)µ-S(CH₂)₂COO⁻Na⁺. In the formula, the symbol "µ" is used to denote a bridging atom. Hence, in the compound Os₃(CO)₁₀(µ-H)µ-S(CH₂)₂COO⁻Na⁺, H and S are bridging atoms.

The metal carbonyl cluster compound may be conjugated or attached to a bioactive agent. As used herein, the term "bioactive agent" is defined as those organic molecules having an effect in a biological system, whether such system is *in vitro, in vivo,* or *in situ.* Biologically active molecules may include growth factors, cytokines, antiseptics, antibiotics, anti-inflammatory agents, analgesics, anesthetics, chemotherapeutic agents, clotting agents, metabolites, chemoattractants, hormones, steroids, and other drugs, or cell attachment molecules.

In various embodiments, the bioactive agent is a protein, an antibody, an antibody fragment, an antibody like molecules, or a drug.

For instance, the bioactive agent may be a proteinaceous molecule, such as an antibody, for example a monoclonal or polyclonal antibody, which immunologically binds to a target analyte at a specific determinant or epitope. The term "antibody" is used in the broadest sense and specifically covers monoclonal antibodies as well as antibody variants, fragments or antibody like molecules, such as for example, Fab, F(ab')₂, scFv, Fv diabodies and linear antibodies, so long as they exhibit the desired binding activity.

In some embodiments, the bioactive agent is a monoclonal antibody. The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they may be synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. The monoclonal antibodies may include "chimeric" antibodies and humanized antibodies. A "chimeric" antibody is a molecule in which different portions are derived from different animal species, such as those having a variable region derived from a murine mAb and a human immunoglobulin constant region.

In some embodiments, the bioactive molecule is a polyclonal antibody. "Polyclonal antibodies" refer to heterogeneous populations of antibody molecules derived from the sera of animals immunized with an antigen, or an antigenic functional derivative thereof. For the production of polyclonal antibodies, host animals such as rabbits, mice and goats, may be immunized by injection with an antigen or hapten-carrier conjugate optionally supplemented with adjuvants.

"Peptide" generally refers to a short chain of amino acids linked by peptide bonds. Typically peptides comprise amino acid chains of about 2-100, more typically about 4-50, and most commonly about 6-20 amino acids. "Polypeptide" generally refers to individual straight or branched chain sequences of amino acids that are typically longer than peptides. "Polypeptides" usually comprise at least about 20 to 1000 amino acids in length, more typically at least about 100 to 600 amino acids, and frequently at least about 200 to about 500 amino acids. Included are homo-polymers of one specific amino acid, such as for example, poly-lysine. "Proteins" include single polypeptides as well as complexes of multiple polypeptide chains, which may be the same or different.

Multiple chains in a protein may be characterized by secondary, tertiary and quaternary structure as well as the primary amino acid sequence structure, may be held together, for example, by disulfide bonds, and may include post-synthetic modifications such as, without limitation, glycosylation, phosphorylation, truncations or other processing.

Antibodies such as IgG proteins, for example, are typically comprised of four polypeptide chains (i.e., two heavy and two light chains) that are held together by disulfide bonds. Furthermore, proteins may include additional components such associated metals (e. g., iron, copper and sulfur), or other moieties. The definitions of peptides, polypeptides and proteins includes, without limitation, biologically active and inactive forms; denatured and native forms; as well as variant, modified, truncated, hybrid, and chimeric forms thereof.

As disclosed herein, the term "drug" generally means a therapeutic or pharmaceutical agent which may be included/mixed into the photoacoustic imaging contrast agent composition, or conjugated or attached to the metal carbonyl cluster compound comprised in the photoacoustic imaging contrast agent composition.

Examples of a drug include: antiproliferative/antimitotic agents including natural products such as vinca alkaloids (e.g. vinblastine, vincristine, and vinorelbine), paclitaxel, epidipodophyllotoxins (e.g. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiproliferative/antimitotic alkylating agents such as nitrogen mustards (such as mechlorethamine, cyclophosphamide and analogs, melphalan, chlorambucil), ethylenimines and methylmelamines (hexamethylmelamine and thiotepa), alkyl sulfonates-busulfan, nirosoureas (carmustine (BCNU) and analogs, streptozocin), trazenes-dacarbazinine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine (cladribine)); platinum coordination complexes (cisplatin, carboplatin), procarbazine, hydroxyurea, mitotane, amino glutethimide; hormones (e.g. estrogen); anticoagulants (heparin, synthetic heparin salts and other inhibitors of thrombin); fibrinolytic agents (such as tissue plasminogen activator, streptokinase and urokinase); antiplatelet (such as aspirin, dipyridamole, ticlopidine, clopidogrel, abciximab); antimigratory; antisecretory (such as breveldin); antiinflammatory: such as adrenocortical steroids (Cortisol, cortisone, fludrocortisone, prednisone, prednisolone, 6-alpha-methylprednisolone, triamcinolone, betamethasone, and dexamethasone), non-steroidal agents (such as salicylic acid derivatives e.g. aspirin); para-aminophenol derivatives (e.g. acetaminophen); indole and indene acetic acids (such as indomethacin, sulindac, and etodalac), heteroaryl acetic acids (such as tolmetin, diclofenac, and ketorolac), arylpropionic acids (such as ibuprofen and derivatives), anthranilic acids (such as mefenamic acid, and meclofenamic acid), enolic acids (such as piroxicam, tenoxicam, phenylbutazone, and oxyphenthatrazone), nabumetone, gold compounds (such as auranofm, aurothioglucose, gold sodium thiomalate); immunosuppressive (such as cyclosporine, tacrolimus (FK-506), sirolimus (rapamycin), azathioprine, mycophenolate mofetil); angiogenic such as vascular endothelial growth factor (VEGF), fibroblast growth factor (FGF); nitric oxide donors; anti-sense oligo nucleotides and combinations thereof.

Use of a photoacoustic imaging contrast agent composition mentioned above in photoacoustic imaging is also disclosed herein.

Radio frequency and optical waves are used in photoacoustic imaging because of their desirable physical properties, such as deeper tissue penetration and better absorption by contrast agents. Advantageously, it is able to provide higher spatial resolutions with high intrinsic contrasts, as compared to most optical imaging techniques. As different biological tissues exhibit different absorption coefficients, by applying photoacoustic imaging on biological samples, measuring the acoustic signals generated with ultrasonic transducers allows rebuilding of the distribution of optical energy deposition, and ultimately to obtain images of the biological tissues.

The photoacoustic imaging may be a laser-based photoacoustic imaging. The photoacoustic imaging is carried out at a wavelength in the visible range of 400 nm to 750 nm. In specific embodiments, the photoacoustic imaging is carried out at a wavelength of about 410 nm.

The photoacoustic imaging is photoacoustic microscopy carried out in a subject. For example, the photoacoustic microscopy may include laser pulse generation, delivery of the laser pulse to a subject under study, reception of photoacoustic signal generated from the subject, image reconstruction, and display of the image. Exemplary illustrations of how photoacoustic microscopy may be carried out are provided in the examples disclosed herein.

Hereinafter, the present invention will be described more fully with reference to the accompanying drawings, in which exemplary embodiments of the invention are shown. This invention may, however, be embodied in many different forms. The exemplary embodiments are provided so that this disclosure will be thorough and complete. In the drawings, lengths and sizes of layers and regions may be exaggerated for clarity.

As used herein, the term "and/or" includes any and all combinations of one or more of the associated listed items. The terminology used herein is for the purpose of describing particular embodiments only. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Although the present invention has been specifically disclosed by preferred embodiments and optional features, modification and variation of the embodiments herein disclosed may be resorted to by those skilled in the art.

The invention has been described broadly and generically herein. Each of the narrower species and subgeneric groupings falling within the generic disclosure also form part of the invention.

Other embodiments are within the following claims and examples. In addition, where features or aspects of the invention are described in terms of Markush groups, those skilled in the art will recognize that the invention is also thereby described in terms of any individual member or subgroup of members of the Markush group.

### EXPERIMENTAL SECTION

In this study, feasibility of osmium carbonyl clusters as a reliable PA contrast agent was explored and its performance in imaging the PA signal changes of the contrast agent at the superior sagittal sinus (SSS) in anaesthetized rat brain was demonstrated. Advantageously, osmium carbonyl clusters are oxygen-stable and moisture-stable. Further, they are suitable for biomedical imaging, and some of their derivatives have potential to interact with various biomolecules.

### Example 1: Synthesis and characterization of compounds

### Example 1.1: General procedures

All manipulations for chemical synthesis were carried out using standard Schlenk techniques under an argon or nitrogen atmosphere. Os₃(CO)₁₂ was purchased from Oxkem; all other chemicals were purchased from other commercial sources and used as supplied. IR spectra were obtained using a Bruker Alpha Fourier transform infrared spectrometer. ¹H NMR spectra were recorded in CDCl₃ on a JEOL ECA400 or ECA400SL spectrometer and were referenced to residual solvent resonances.

### Example 1.2: Synthesis of Os₃(CO)₁₀(µ-H)µ-S(CH₂)₂COOH

Os₃(CO)₁₀(NCCH₃)₂ (200 mg, 0.22 mmol) was stirred with thioglycolic acid (48 mg, 0.22 mmol) in dichloromethane (30 ml) at room temperature for 12 h. After the reaction, the solvent was removed in vacuo and the residue purified by column chromatography using hexane/DCM as the eluant to yield a yellow solid. Yield: 57%.
IR (CH₂Cl₂): v_{CO} 2106 (w), 2066 (s), 2057(s), 2020 (s), 1997 (w).
¹H NMR (CDCl₃): δ 2.58-2.69 (m, 4H, C*H*₂C*H*₂), -17.40 (s, 1H, Os*H*Os)

### Example 1.3: Synthesis of Os₃(CO)₁₀(µ-H) µ-S(CH₂)₂COO⁻Na⁺ (2)

To OS₃(CO)₁₀(µ-H)µ-S(CH₂)₂COOH (100 mg, 0.09 mmol) in methanol (20 ml) was added sodium carbonate (18.7 mg, 0.19 mmol). The mixture was refluxed for 1 h, cooled to room temperature, and the excess sodium carbonate filtered off. The solvent was removed under reduced pressure to afford **2** as a water-soluble, yellow solid. Yield: 95%.
IR (KBr): v_{CO} 2110 (w), 2061 (s), 2009 (s), 1928 (w).
¹H NMR (300 MHz, D₂O): δ 2.47-2.69 (m, 4H, C*H*₂C*H*₂)*,* -17.11 (s, 1H, Os*H*Os) ESI (m/z): 957.1 [M⁻]

### Example 2: Dark-field confocal photoacoustic microscopy system and experimental set-up

The 50-MHz dark-field confocal fPAM system used and the experimental set-up for *in vivo* imaging, is depicted below.

The microscopy system consists of laser pulse generation and delivery, PA signal reception, and image reconstruction and display. Laser pulses, 4 ns wide, were generated at a frequency of 10 Hz by using an optical parametric oscillator (Surlite OPO Plus, Continuum, USA). The laser was pumped by a frequency-tripled Nd:YAG Q-switched laser (Surlite II-10, Continuum, USA). The incident wavelength was set at 410 nm. The custom-made 50-MHz ultrasonic transducer (Acoustic Sensor, Taiwan) has a -6 dB fractional bandwidth of 57.5%, focal length of 9 mm, and a 6 mm active element, offering an axial resolution of 32 µm and a lateral resolution of 61 µm.

Laser energy was delivered using a 1-mm multimodal fiber. The fiber tip was coaxially aligned with a convex lens, an axicon, a plexiglass mirror, and an ultrasonic transducer on an optical bench, forming dark-field illumination that was confocal with the focal point of the ultrasonic transducer. The incident energy density on the sample surface was well within American National Standards Institute (ANSI) safety limits. The transducer was immersed in an acrylic water tank during the imaging process, and the hole at the bottom of the tank was sealed with a piece of 15-µm thick polyethylene film. A thin layer of ultrasonic gel was applied as a PA conductive medium, which was then attached to the thin polyethylene film to ensure reliable coupling of the PA waves with the water tank. The PA signals received by the ultrasonic transducer were pre-amplified by a low-noise amplifier (noise figure 1.2 dB, gain 55 dB, AU-3A-0110, USA), cascaded to an ultrasonic receiver (5073 PR, Olympus, USA) and then digitized and sampled by a computer-based 14-bit analog to digital (A/D) card (CompuScope 14220, GaGe, USA) at a 200-MHz sampling rate for data storage.

Fluctuations in the laser energy were monitored with a photodiode (DET36A/M, Thorlabs, USA). The recorded photodiode signals were measured prior to the experiment to compensate for PA signal variations caused by laser-energy instability. The achievable penetration depth of the current PA microscopy system was 3 mm with approximately 18-dB SNR, where SNR is defined as the ratio of the signal peak value to the root-mean-square value of the noise. Three scan types can be provided by this system: A-line (i.e., one-dimensional images where the axis represents the imaging depth), B-scan (i.e., two-dimensional images where one axis is the lateral scanning distance and the other is the imaging depth), and C-scan (i.e., projection images from the three-dimensional images). The amplitude of the envelope-detected PA signals was used in the subsequent functional imaging analysis.

For both *in vivo* and *in vitro* imaging, the laser was pulsed with a pulse repetition rate of 10 Hz and coupled by a lens to an optical fiber to illuminate the ROI. A window at the bottom of the water container was sealed with an optically and ultrasonically transparent, disposable polyethylene film. After a commercially available ultrasound gel was applied to the brain for acoustic coupling, the brain was placed between the water container and the custom-made stereotaxic apparatus for imaging.

### Example 3: PA study of trinuclear carbonyl clusters

The trinuclear carbonyl clusters of the group 8 metals, *viz.,* M₃(CO)₁₂ (**1**, M = Fe (**a**), Ru (**b**) or Os(**c**)) are all readily available and variedly coloured.

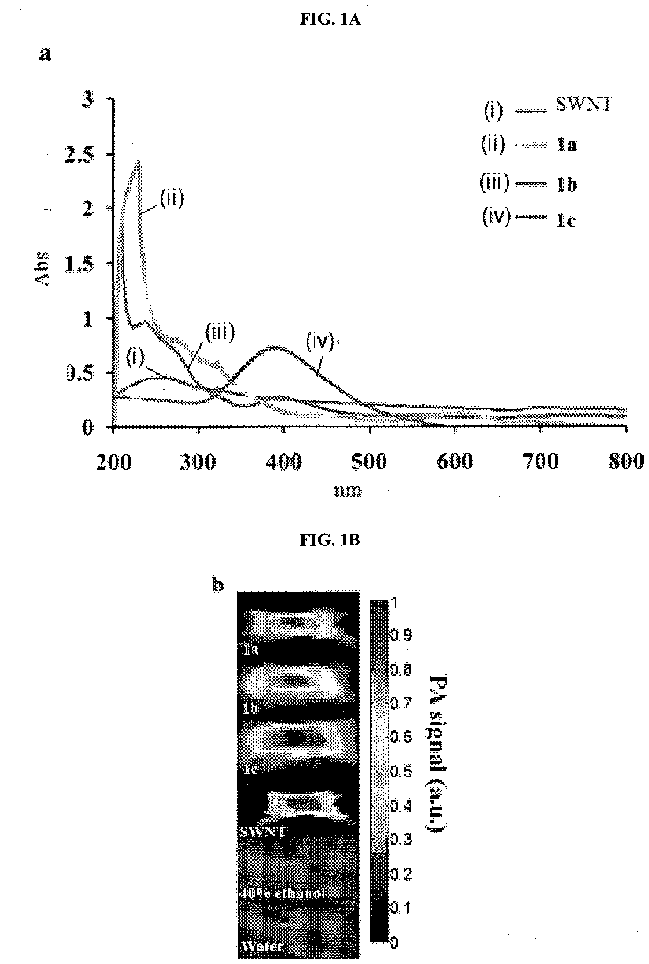

**FIG. 1A** shows UV-vis spectra of organometallic compounds M₃(CO)₁₂ (M = Fe, Ru or Os, **1 a-c**) used for PA study. Single-walled carbon nanotubes (SWNT) were used for comparison. **FIG. 1B** shows false color images representing the relative PA signal strengths.

Consistent with their electronic spectra, **1c** exhibited the highest PA signal among the three carbonyl clusters (**FIG. 1**); it was about 1.5 and 1.2 times higher than that for **1a** and **1b,** respectively, and more significantly, 2.4 times higher than that for single-walled carbon nanotubes (SWNT). The absorbance maximum at about 380 nm for **1c,** for example, has been assigned to a metal-metal bond σ to σ* transition.

All the PA images in this study were acquired at 410 nm without any need for signal averaging, and yet the contrast quality of the PA images is high.

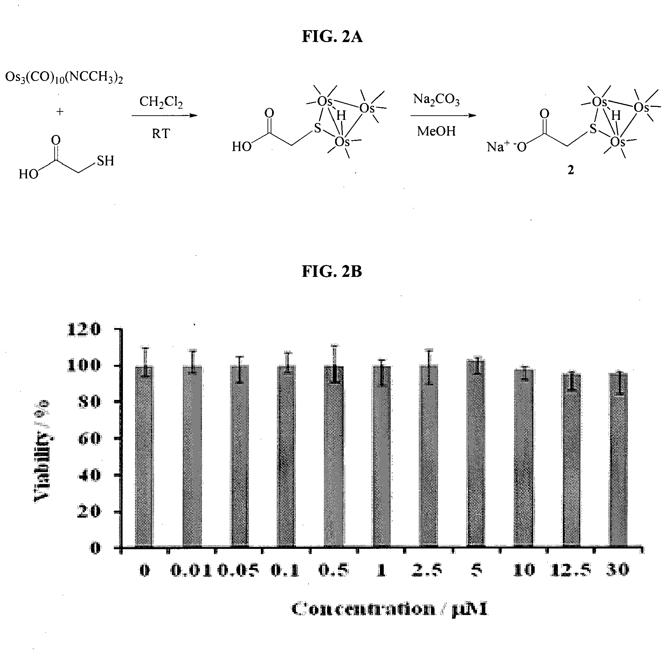

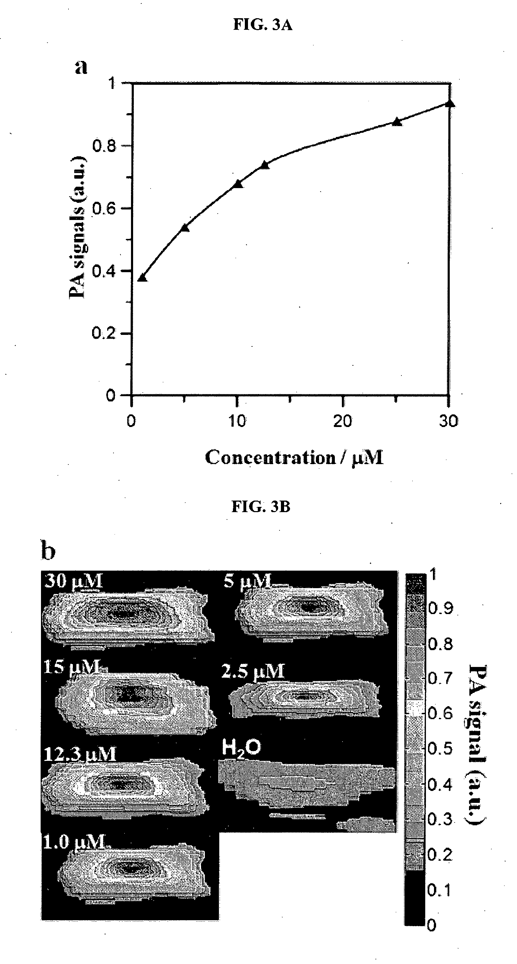

The compounds **1 a-c** were dissolved in a water/ethanol (60:40, v/v) mixture. The compound **1c**, however, has low water solubility. The water-soluble cluster [Os₃(CO)₁₀(µ-H)(µ-S(CH₂)₂COO)]⁻Na⁺ (**2**) was thus synthesized, and its cytoxicity was evaluated. An MTS cytotoxicity assay shows that **2** poses little effect on the viability of cells (**FIG. 2**). The PA signal for an aqueous solution of **2** was found to be adequate even down to 2.5 µM (**FIG. 3**).

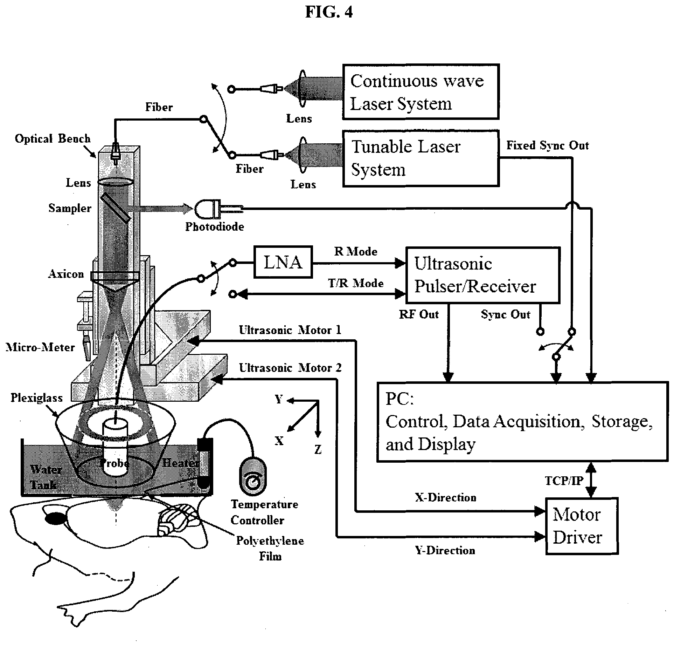

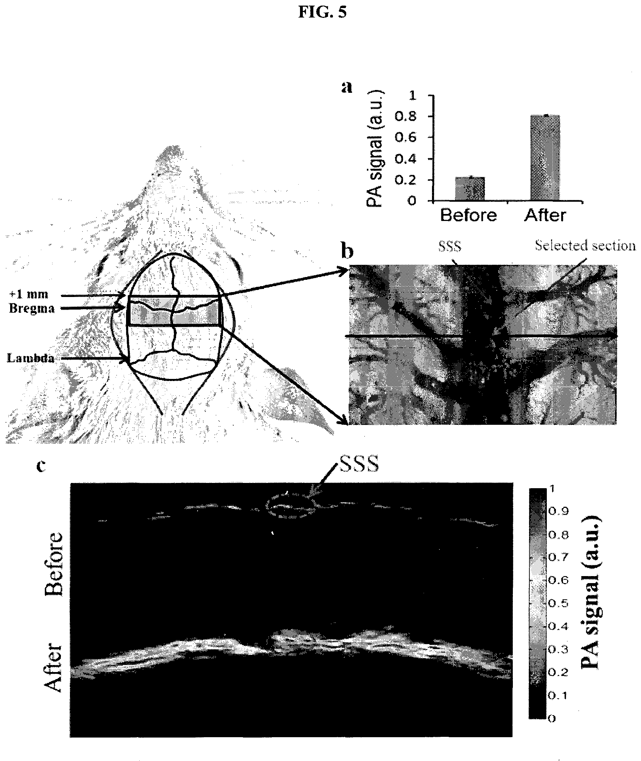

Cortical vasculature of the rat was *in vivo* imaged at 32 × 61 µm resolution, with a designed 50-MHz dark-field confocal PA microscopy system (**FIG. 4**); the PA signal at λ₄₁₀ was acquired. The SSS is the largest vein in the rodent brain cortex, and a selected section of it is the region-of-interest (ROI) in this study (**FIG. 5B**). There is strong clinical evidence that controlling cerebral venous blood flow is critically important for patients with severe traumatic brain injury as well as for patients undergoing cardiac surgery. Then, the SSS is a large central cerebral vein at cortical layer, and collects blood flow from both hemispheres before emptying into the internal jugular vein. That is, accurate measurements of blood flow changes in the SSS are thus very important.

Some of the optical techniques can monitor intrinsic changes of SSS based on detection of light diffusively scattered from a target tissue, like Near-infrared spectroscopy (NIRS). However, NIRS measures only volume-averaged of brain tissue and cannot distinguish between venous, capillary, and arterial blood. In contrast, PA imaging can see venous, capillary, and arterial blood. However, it was found that the SSS was not satisfactorily visualized in the projected PA C-scan image. Although the SSS could be seen in the PA B-scan images, the detected PA signals from the SSS were weaker than those from other cortical vessels. This weaker SSS signals may result from the frequency of the PA signals generated by the SSS may be out of the detection bandwidth of the transducer. Moreover, sensing with spherical geometric focusing inherently prefers point-like PA sources, which is not the case of for the large sized SSS, thus weakening the detection of PA signals from the SSS. That is, probing the changes of SSS via the designed contrast agents provide a novel insight for optical imaging technique to study the SSS related neuroscience issues. Compared to the brain PA B-scan image obtained from the intrinsic optical contrast, the image acquired 20 min after the administration of a solution of **2** showed the brain vasculature with greater clarity, especially the SSS (**FIG. 5C**).

### Example 4: Cell viability study

Five thousand oral squamous cell carcinoma (OSCC) cells were seeded in a 96-well plate for 24 h before a solution of **2** in DMEM (1 mL) with the indicated concentrations (0.01 - 30 µM) was introduced into each well. After incubation for 24 h, 10 µL of CCK8 (cell counting kit-8, Sigma-Aldrich) was added to each well. Cell absorbance was measured with a SpectraMax 384 Plus spectral analyser after 4 h, with 450 nm excitation.

### Example 5: In vitro measurements

A polyethylene tubing (about 20 cm in length) was filled with the sample solution and then positioned at the focus of the transducer, i.e. at a depth of 9 mm with respect to the transducer in the water tank. The system was maintained in a 25 °C water bath throughout the experiment. Imaging was carried out with the PA microscopy system at 32 × 61-µm resolution, with a scanning step size of 20 µm for each B-scan.

### Example 6: In vivo measurements

Six male Wistar rats (NUS-CARE, Singapore), weighing 250 - 300 grams, were used. Animal experiments were conducted in accordance with guidelines from Institutional Animal Care and Use Committee (IACUC) at the National University of Singapore. The rats were fasted for 24 h prior to the imaging experiments, but were given water ad libitum. They were anesthetized with isoflurane (2 to 3 % in 100 % O₂), mounted in a dorsal position over a custom-made acrylic stereotaxic holder, and the skin and muscle on the skull were removed to expose the bregma landmark. The anteroposterior (AP) distance between the bregma and the interaural line was directly surveyed. The bregma was 9.3 ± 0.12 mm (mean ± standard deviation [SD]) anterior to the interaural line.

A craniotomy was also performed for each animal, and a bilateral cranial window of approximately 6 (horizontal) × 4 (vertical) mm size was made with a high-speed drill. After the rat was secured to the stereotaxic frame and placed on the bed pallet, the pallet was moved to position at the bregma, which was 9 mm anterior to an imaginary line drawn between the centers of each ear bar (the interaural line). The interaural and bregma references were then used to position the heads in the PAM system. After bregma positioning, a PA C-scan (projection image from the three-dimensional images) was performed to acquire reference images of the cortical vasculature, at λ₄₁₀. 200 µL of **2** at 30 µM was administered via retro-orbital injection prior to imaging.

We have shown in this study that organometallic carbonyl cluster compounds may be safe contrast agents for use in laser-based photoacoustic imaging. As demonstrated herein, a water-soluble osmium carbonyl cluster was used for the *in vivo* imaging of the rat cerebral vasculature, with high spatial resolution and satisfactory sensitivity. Use of an exogenous contrast agent may potentially provide a method to monitor intra- or extravascular blood flow in biological tissues. This is important for an accurate assessment of acute ischaemic stroke, lesion development, thermal injury, neovascularization, tumor angiogenesis, tumor necrosis, hepatic function and regional blood flow response. Choice of the osmium carbonyl clusters rests primarily because on their well-studied chemistry, and also on the fact that they offer multiple sites for attachment of various ligands as property modifiers. Through conjugation to bioactive materials such as proteins, antibodies, and drugs, molecular imaging and therapeutic monitoring may also be carried out.

## Claims

1. Use of a photoacoustic imaging contrast agent composition in photoacoustic microscopy carried out in a subject at a wavelength in the visible range of 400 nm to 750 nm, the composition comprising a metal carbonyl cluster compound having the general formula (I)
M₃(CO)ₓL₁₂₋ₓ (I)
wherein
M at each occurrence denotes a metal independently selected from the group consisting of Fe, Ru, and Os;
x is an integer from 10 to 12; and
each L is independently selected from the group consisting of -H and -A-(CH₂)ₙ-COO⁻Y⁺, wherein A is selected from the group consisting of S, O, C and N; n is an integer from 1 to 10; and Y is any cation.

2. The use according to claim 1, wherein M is Os.

3. The use according to claim 1, wherein x is 10.

4. The use according to claim 1, wherein A is S.

5. The use according to claim 1, wherein n is an integer from 1 to 6.

6. The use according to claim 1, wherein n is 2.

7. The use according to claim 1, wherein Y is selected from the group consisting of an alkali metal and NH₄.

8. The use according to claim 1, wherein Y is selected from the group consisting of Na and K.

9. The use according to claim 1, wherein Y is Na.

10. The use according to claim 1, wherein the metal carbonyl cluster compound is O_{S3}(CO)₁₀(µ-H)µ-S(CH₂)₂COO⁻Na⁺.

11. The use according to claim 1, wherein the metal carbonyl cluster compound is conjugated or attached to a bioactive agent.

12. The use according to claim 11, wherein the bioactive agent is selected from the group consisting of protein, antibody, antibody fragment, antibody like molecule, or drug.

13. The use according to claim 1, wherein the photoacoustic microscopy is carried out at a wavelength of about 410 nm.

## Patentansprüche

1. Verwendung einer photoakustischen Bildgebungskonstrastmittelzusammensetzung in der photoakustischen Mikroskopie, die in einem Subjekt bei einer Wellenlänge im sichtbaren Bereich von 400 nm bis 750 nm durchgeführt wird, wobei die Zusammensetzung eine Metallcarbonylcluster-Verbindung umfasst, die die allgemeine Formel (I) aufweist
M₃(CO)ₓL₁₂₋ₓ (I)
wobei
M bei jedem Auftreten ein Metall bezeichnet, unabhängig ausgewählt aus der Gruppe bestehend aus Fe, Ru und Os;
x eine ganze Zahl von 10 bis 12 ist; und
jedes L unabhängig ausgewählt ist aus der Gruppe bestehend aus -H und -A-(CH₂)ₙ-COO⁻Y⁺, wobei A ausgewählt ist aus der Gruppe bestehend aus S, O, C, und N; n eine ganze Zahl von 1 bis 10 ist; und
Y ein beliebiges Kation ist.

2. Die Verwendung gemäß Anspruch 1, wobei M gleich Os ist.

3. Die Verwendung gemäß Anspruch 1, wobei x gleich 10 ist.

4. Die Verwendung gemäß Anspruch 1, wobei A gleich S ist.

5. Die Verwendung gemäß Anspruch 1, wobei n eine ganze Zahl von 1 bis 6 ist.

6. Die Verwendung gemäß Anspruch 1, wobei n gleich 2 ist.

7. Die Verwendung gemäß Anspruch 1, wobei Y ausgewählt ist aus der Gruppe bestehend aus einem Alkalimetall und NH₄.

8. Die Verwendung gemäß Anspruch 1, wobei Y ausgewählt ist aus der Gruppe bestehend aus Na und K.

9. Die Verwendung gemäß Anspruch 1, wobei Y gleich Na ist.

10. Die Verwendung gemäß Anspruch 1, wobei die Metallcarbonylcluster-Verbindung gleich Os₃(CO)₁₀(µ-H)µ-S(CH₂)₂COO⁻Na⁺ ist.

11. Die Verwendung gemäß Anspruch 1, wobei die Metallcarbonylcluster-Verbindung an ein bioaktives Mittel konjugiert oder angehängt ist.

12. Die Verwendung gemäß Anspruch 11, wobei das bioaktive Mittel ausgewählt ist aus der Gruppe bestehend aus Protein, Antikörper, Antikörperfragment, Antikörper-ähnlichem Molekül oder Wirkstoff.

13. Die Verwendung gemäß Anspruch 1, wobei die photoakustische Mikroskopie bei einer Wellenlänge von etwa 410 nm durchgeführt wird.

## Revendications

1. Utilisation d'une composition d'agent de contraste d'imagerie photoacoustique dans une microscopie photoacoustique réalisée sur un sujet à une longueur d'onde dans la plage du visible de 400 nm à 750 nm, la composition comprenant un composé métallique à groupement carbonyle ayant la formule générale (I)
M₃(CO)ₓL₁₂₋ₓ (I)
dans laquelle
M dans chaque cas représente un métal indépendamment choisi dans le groupe constitué par Fe, Ru et Os ;
x est un nombre entier de 10 à 12 ; et
chaque L est indépendamment choisi dans le groupe constitué par -H et -A-(CH₂)ₙ-COO⁻Y⁺, où A est choisi dans le groupe constitué par S, O, C et N ; n est un nombre entier de 1 à 10 ; et Y est un quelconque cation.

2. Utilisation selon la revendication 1, dans laquelle M est Os.

3. Utilisation selon la revendication 1, dans laquelle x est 10.

4. Utilisation selon la revendication 1, dans laquelle A est S.

5. Utilisation selon la revendication 1, dans laquelle n est un nombre entier de 1 à 6.

6. Utilisation selon la revendication 1, dans laquelle n est 2.

7. Utilisation selon la revendication 1, dans laquelle Y est choisi dans le groupe constitué par un métal alcalin et NH₄.

8. Utilisation selon la revendication 1, dans laquelle Y est choisi dans le groupe constitué par Na et K.

9. Utilisation selon la revendication 1, dans laquelle Y est Na.

10. Utilisation selon la revendication 1, dans laquelle le composé métallique à groupement carbonyle est Os₃(CO)₁₀(µ-H)µ-S(CH₂)₂COO⁻Na⁺.

11. Utilisation selon la revendication 1, dans laquelle le composé métallique à groupement carbonyle est conjugué ou attaché à un agent bioactif.

12. Utilisation selon la revendication 11, dans laquelle l'agent bioactif est choisi dans le groupe constitué par une protéine, un anticorps, un fragment d'anticorps, une molécule de type anticorps ou un médicament.

13. Utilisation selon la revendication 1, dans laquelle la microscopie photoacoustique est réalisée à une longueur d'onde d'environ 410 nm.
